Europäisches Patentamt

European Patent Office

Office européen des brevets

(19)

(11) Veröffentlichungsnummer: **0 150 792**
**B1**

(12) **EUROPÄISCHE PATENTSCHRIFT**

(45) Veröffentlichungstag der Patentschrift: **23.01.91**

(21) Anmeldenummer: **85100585.0**

(22) Anmeldetag: **21.01.85**

(51) Int. Cl.⁵: **A 61 K 33/06,** A 61 K 33/08, A 61 K 33/10

(54) **Arzneimittel und die Verwendung schwerlöslicher Calcium- und/oder Magnesiumverbindungen zur Herstellung eines Arzneimittels.**

(30) Priorität: **27.01.84 DE 3402878**

(43) Veröffentlichungstag der Anmeldung:
**07.08.85 Patentblatt 85/32**

(45) Bekanntmachung des Hinweises auf die Patenterteilung:
**23.01.91 Patentblatt 91/04**

(84) Benannte Vertragsstaaten:
**AT BE CH DE FR GB IT LI NL SE**

(56) Entgegenhaltungen:
**WO-A-84/03623**
**DE-A-2 132 923**
**DE-A-2 618 083**
**FR-A-2 279 414**
**FR-M- 4 265**

**Martindale, The Extra Pharmacopoeia, 28. Ausgabe 1982, S. 82 und 83, 699t bzw. 702-r**

(73) Patentinhaber: **Algina Aktiengesellschaft c/o Rensch Treuhand Baarer Strasse 43 CH-6301 Zug (CH)**

(72) Erfinder: **Dietl, Hans, Dr. Chem. Eichendorffstrasse 33 D-8202 Bad Aibling (DE)**

(74) Vertreter: **Kraus, Walter, Dr. et al Patentanwälte Kraus, Weisert & Partner Thomas-Wimmer-Ring 15 D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Arzneimittel zur Behandlung der Hyperphosphatämie bei chronischer Urämie und zur Behandlung von Dialyse-Patienten sowie zur Prävention von Nierensteinen, insbesondere phosphat- und oxalathaltigen Nierensteinen.

Die Erfindung betrifft weiterhin die Verwendung schwerlöslicher Calcium- und/oder Magnesiumverbindungen zur Herstellung eines Arzneimittels zur Bekämpfung der Hyperphosphatämie und zur Prävention von Nierensteinen.

In der Bundesrepublik Deutschland gibt es zur Zeit ca. 5000 Patienten mit chronischer Urämie (=Niereninsuffizienz) sowie etwa 15 000 Patienten, bei denen diese Urämie so weit fortgeschritten ist, daß eine Blutwäsche mittels künstlicher Nieren, das heißt eine Dialysebehandlung, notwendig ist. Diese Patienten müssen sich bis zu dreimal wöchentlich für drei bis sechs Stunden einer Blutwäsche nach einem der bekannten Verfahren, wie beispielsweise Hämodialyse, Hämofiltration, Peritonealdialyse oder kontinuierlicher ambulanter Peritonealdialyse, unterziehen, um urämische Toxine, wie z.B. Harnstoff, Harnsäure, Creatinin etc., Kalium sowie Phosphat aus dem Organismus, das heißt aus dem Blut, zu entfernen.

Trotz zusätzlicher diätetischer Maßnahmen, wie z.B. diätetischer Begrenzung der Phosphatzufuhr durch die Nahrung, ist jedoch der Phostphatspiegel bei den meisten Patienten auch nach dieser Blutreinigung noch zu hoch. Die Hyperphosphatämie ist ein wesentlicher Faktor in der Pathogenese des sekundären Hyperparathyseodismus sowie bei der Entwicklung der renalen Osteopathie unter chronischer Dialysebehandlung. Deshalb sind fast alle Urämiker auf eine zusätzliche Medikation zur Senkung des überhöhten Phosphatspiegels in den Normbereich angewiesen.

In den letzten Jahren wurden überwiegend Aluminiumhydroxide, wie z.B. Aludrox®, Anti-Phosphat oder Aluminiumhydroxycarbonate, zur intestinalen Phosphatbindung und zur Senkung überhöhter Serumphosphatspiegel verwendet. Dabei wurden bis zu 10 g Medikament und mehr pro Tag verabreicht [M. B. Kaye, Arch. Intern. Med. *124*, 656 (1969)]. In der DE—OS 26 18 083 wird ein weiteres aluminiumhaltiges Salz zur Reduktion der Phosphatresorption beschrieben.

Diesen bekannten Mitteln zur Phosphatbindung haften jedoch Nachteile an. Durch partielle Lösung des Aluminiumhydroxids bzw. Aluminiumhydoxidcarbonats im Magenmilieu zu Aluminiumhydroxidchlorid und Aluminiumchlorid wird $Al^{3\oplus}$ freigesetzt, welches im Magen und vor allem im oberen Dünndarm resorbiert wird und über das Blut sowohl in die Knochensubstanz als auch in andere Teile des Körpers gelangt und bei langjährigem Einwirken erhebliche Schäden hervorrufen kann, z.B. Dialyse-Enzephalopathie, Osteomalazie mit Neigung zu Spontan-Frakturen, Anämie etc. [A. C. Alfrey et al, Trans. Am. Soc. Artif. Int. Org. *18*, 257 (1972); A. C. Alfrey et al, Kidney Intern. *18*, 115 (1980), J. T. McCall et al, Kidney Intern. *18*, 115 (1980)]. Laut Statistik sterben von jährlich 10 bis 12% der Dialyse-Patienten mit zerebralen Zwischenfällen nicht wenige an einer Dialyse-Enzephalopathie [H. Pogglitsch, Nieren und Hochdruckkrankheiten *10*, 210 (1981)].

Auch bei Verabreichung von Aluminiumhydroxid in magensaftresistenter, jedoch dünndarmlöslicher Form kommt es immer noch zu einer unerwünschten Resorption von Aluminium [H. Pogglitsch et al, Nieren und Hochdruckkrankheiten, *12*, 186 (1983)]. Ein Absetzen der zur Zeit verwendeten aluminiumhaltigen Präparate zur Phosphatbindung erscheint daher geboten.

Neben aluminiumhaltigen Präparaten wurde zur Phosphatsenkung in Einzelfällen auch Calciumcarbonat verwendet. Calciumcarbonat löst sich im sauren Milieu des Magens, im neutralen Milieu des Dünndarms kann dann Phosphat als unlösliches Calciumphosphat gebunden und mit dem Stuhl ausgeschieden werden. Diese Therapie hat jedoch schwerwiegende Probleme, es kommt zu einer verstärkten Aufnahme von Calcium in das blut und schließlich zu einer akut lebensbedrohlichen Hypercalciämie. Daher hat sich diese Therapie mit Calciumcarbonat bisher nicht durchgesetzt.

Phosphathaltige Nierensteine können auch durch die Aufnahme von Phosphat durch die Nahrung gebildet werden, wobei sich schließlich vorwiegend Calciumphosphat-Steine bilden. Zur Prävention des Wiederauftretens von phosphathaltigen Nierensteinen werden ebenfalls aluminiumhaltige Phosphatbinder mit den vorher genannten Nachteilen verwendet.

Dementsprechend wird durch die Aufnahme oxalathaltiger und oxalatreicher Nahrung die Entstehung oxalathaltiger Nierensteine gefödert.

In der FR—A—4 265 M (vgl. Résumé 3° und 4°) werden magensaftresistente Arzneimittel beschrieben, die als wesentlichen Bestandteil, entweder Magnesiumhydroxid oder Magnesiumoxid enthalten.

In der Literaturstelle Martindale (The Extra Pharmacopoeia), 28. Auflage 1982, Seiten 82 und 83 und insbesondere 699-t, wird die Verwendung von Magnesiumhydroxid und leichtem Magnesiumoxid als Arzneimittel beschrieben. Hinweise, daß bestimmte Calciumverbindungen zur Behandlung der Hyperphosphatämie und zur Prävention phosphat- und/oder oxalathaltiger Nierensteine verwendet werden können, finden sich in dieser Literaturstelle nicht.

Der Erfindung liegt die Aufgabe zugrunde, ein Arzneimittel zur Verfügung zu stellen, mit dem die Aufnahme von Phosphat, insbesondere bei chronischer Urämie und bei Dialysepatienten, vermindert und damit die Serumphosphatspiegel gesenkt werden können. Erfindungsgemäß sollen insbesondere Arzneimittel zur Verfügung gestellt werden, mit denen die Bildung von phosphat- und/oder oxalathaltigen Nierensteinen verhindert werden kann. Dieses Mittel soll insbesondere Patienten verabreicht werden, von

2

## EP 0 150 792 B1

denen bekannt ist, daß sie zur Nierensteinbildung niegen. Das Mittel soll die Nachteile der im Handel befindlichen Mittel nicht aufweisen.

Erfindungsgemäß soll weiterhin die Verwendung schwerlöslicher Calcium- und/oder Magnesiumverbindungen zur Herstellung eines Arzneimittels mit einer neuen Indikation zur Verfügung gestellt werden.

Gegenstand der Erfindung ist ein Arzneimittel zur Behandlung der Hyperphosphatämie und zur Prävention phosphat- und/oder oxalathaltiger Nierensteine, das dadurch gekennzeichnet ist, daß es eine bei pH-Werten von 6 bis 9 schwerlösliche Calciumverbindung, nämlich Calciumcarbonat, Calciumhydroxid, Calciumoxid und/oder Calciumsulfat, gegebenenfalls zusammen mit üblichen Träger- und/oder Verdünnungsstoffen in magensaftresistenter Arzneiform enthält.

Gegenstand der Erfindung ist weiterhin die Verwendung von mindestens einer bei pH-Werten von 6 bis 9 schwerlöslichen Calcium- und/oder Magnesiumverbindung zur Herstellung eines Arzneimittels in magensaftresistenter Arzneiform, zur Bekämpfung der Hyperphosphatämie und zur Prävention phosphat- und/oder oxalathaltiger Nierensteine.

Die Erfindung betrifft ebenfalls die Verwendung von

i) Calciumcarbonat zur Herstellung eines Arzneimittels in magensaftresistenter Form zur Bekämpfung der Hyperphosphatämie und zur Behandlung phosphathaltiger Nierensteine,

ii) Magnesiumcarbonat, Magnesiumhydroxid-Carbonat, Magnesiumhydroxid und/oder Magnesiumoxid zur Herstellung eines Arzneimittels in magensaftresistenter Form zur Behandlung oxalathaltiger Nierensteine, und

iii) Calcium- und Magnesiumverbindungen, die bei pH-Werten von 6 bis 9 schwerlöslich sind, gemeinsam, zur Herstellung eines Arzneimittels in magensaftresistenter Form zur Bekämpfung oxalathaltiger Nierensteine.

Es wurde überraschenderweise gefunden, daß bei Verabreichung von bei pH-Werten von 6 bis 9 schwerlöslichen Calcium- und/oder Magnesiumverbindungen eine gute Bindung von Phosphaten bzw. Oxalaten in Form von unlöslichem Calciumphosphat bzw. Calciumoxalat oder Magnesiumphosphat bzw. Magnesiumoxalat kommt, ohne daß es zu einer übermäßigen unerwünschten Resorption von Calcium- bzw. Magnesiumkationen im Blut kommt, wie dies z.B. bei Verabreichung von löslichen Calcium- und Magnesiumsalzen, wie z.B. den Citraten oder bei Carbonaten, die sich im Magen lösen, auftritt.

Es wurde weiterhin überraschenderweise gefunden, daß die gewünschte Wirkung erhalten wird, wenn die Calcium- und Magnesiumverbindungen in magensaftresistenten Arzneiformen verwendet werden.

Entscheidend ist bei der Erfindung, daß die Verabreichung in der magensaftresistenten Arzneiform erfolgt. Diese Art der Verabreichung ermöglicht völlig überraschend und unerwartet die Bindung von Phosphat bzw. Oxalat, ohne eine übermäßige störende Resorption von Calcium bzw. Magnesium ins Blut, wie diese bei der bisherigen Form der Verabreichung dieser Calcium- bzw. Magnesiumverbindungen eintrat.

Die magensaftresistente Arzneiform setzt im Dünndarm bei pH-Werten von 6 bis 8 die schwerlösliche Calcium- und Magnesiumverbindung frei, welche langsam mit dem im Dünndarm vorhandenen Phosphat und/oder Oxalat zu unlöslichen Calciumphosphaten und Calciumoxalaten bzw. Magnesiumphosphaten und Magnesiumoxalaten reagiert. Diese werden dann ohne Resorption im Stuhl ausgeschieden.

Da das Arzneimittel, z.B. Calcium- oder Magnesiumcarbonat, bei den im Dünndarm herrschenden pH-Werten von 6 bis 8 in schwerlöslicher Form vorliegt, kommt es im Gegensatz zur Verabreichung in der üblichen magensaftlöslichen Arzneiform nicht zu einer übermäßigen Resorption von Calcium- bzw. Magnesiumionen.

In dem erfindungsgemäßen Arzneimittel und bei de erfindungsgemäßen Verwendung werden bei pH-Werten von 6 bis 9, vorzugsweise von 6,5 bis 8, schwerlösliche Calcium- und/oder Magnesiumverbindungen verwendet. Beispiele hierfür sind Calciumcarbonat, Calciumhydroxid, Calciumoxid, Calciumsulfat, Magnesiumcarbonat, Magnesiumhydroxidcarbonat, Magnesiumoxid und Magnesiumhydroxid. Selbstverständlich kann man auch Gemische aus mindestens zwei oder mehr dieser Verbindungen verwenden.

Erfindungsgemäß ist es bevorzugt, daß Calciumcarbonat zur Behandlung der Hyperphosphatämie und zur Behandlung phosphathaltiger Nierensteine verwendet wird. Es ist weiterhin bevorzugt, die Magnesiumverbindungen, vorzugsweise Magnesiumcarbonat, Magnesiumhydroxidcarbonat, Magnesiumhydroxid und Magnesiumoxid oder ihre Gemische zur Behandlung oxalathaltiger Nierensteine zu verwenden. Gemische aus den oben erwähnten Calcium- und Magnesiumverbindungen eignen sich weiterhin zur Behandlung oxalathaltiger Nierensteine.

Die Calcium-, Magnesiumverbindungen können gegebenenfalls zusammen mit üblichen Arzneimittelträgerstoffen und Verdünnungsmitteln in Form magensaftresistenter Arzneiformen eingesetzt werden. Diese können in an sich bekannter Weise in Form von magensaftresistenten Tabletten, magensaftresistenten Hartgelatinekapseln, magensaftresistenten Weichgelatinekapseln oder magensaftresistenten Granulaten vorliegen. Die Verbindungen können weiterhin in magensaftresistenter Retardform vorliegen, so daß nur eine einmalige Gabe pro Tag erforderlich ist.

Die in dem erfindungsgemäßen Arzneimittel vorhandenen und die erfindungsgemäß zu verwendenden Calcium- und Magnesiumverbindungen sind im Handel erhältlich. Sie können zur Herstellung der erfindungsgemäßen Arzneimittel in Form von Pulvern, Granulaten, Perlen etc. verwendet

3

EP 0 150 792 B1

werden. Die Pulver können in feiner oder grober Form vorliegen.

Die Phosphatbindung beträgt z.B. bei Calciumcarbonat in vitro ca. 40 bis 180 mg/g, bei Calciumsulfat ca. 100 bis 300 mg/g und ist damit höher als die vieler Aluminiumhydroxide.

Die Oxalatbindung beträgt für Calciumcarbonat ca. 50 bis 200 mg Oxalat/g, bei Calciumsulfat ca. 100 bis 250 mg Oxalat/g. Für die Magnesiumverbindungen werden ähnliche Werte festgestellt.

Es war überraschend und hat nicht nahegelegen, daß die Phosphatbindungskapazität von den Calcium- und Magnesiumverbindungen in der gleichen Größenordnung liegt wie die des Aluminiumhydroxids und teilweise noch höher ist.

Die folgenden Beispiele erläutern die Erfindung. In den Beispielen wurden die folgenden Bestimmungsmethoden verwendet.

a) Bestimmung von $HPO_4^{2\ominus}$

$HPO_4^{2\ominus}$ wurde nach der Methode von Gomorri [G. Gomorri, J. Lab. Clin. Med. *27*, 955 (1942)] photometrisch bestimmt. Dabei bildet sich anorganisches Phosphat mit Natriummolybdatphosphormolybdat, das durch Reduktion mit p-Methylaminophenolsulfat in kolloidales Molybdänblau überführt wird, welches photometrisch bestimmt wird.

b) Bestimmung von Oxalat

Die titrimetrische Bestimmung von Oxalat erfolgt mit einer 0.02 Kaliumpermanganat-Meßlösung, die zur Stabilisierung 1 Stunde gekocht worden war. 10 ml der Probe wurden mit 150 ml bidestilliertem Wasser und 10 ml einer 1+4 verdünnten konzentrierten Schwefelsäure versetzt, auf 75 bis 85°C ernitzt und bis zu einer bleibenden schwachen Rosäfarbung mit der $KMnO_4$-Lösung titriert.

Beispiel 1

Es wurden in an sich bekannter Weise magensaftresistente Tabletten bzw. magensaftresistente Hartgelatinekapseln hergestellt mit je 500 mg Arzneistoff.

Mit Tris-Puffer, Natronlauge und Überschuß von $K_2HPO_4$ wurde auf den gewünschten pH-Wert eingestellt, die Tablette bzw. Hartgelatinekapsel zugegeben und 1 Stunde inkubiert. Dann wurde das freie Phosphat bestimmt und damit das Phosphatbindevermögen bestimmt.

Folgende Ergebnisse wurden erhalten:

Calciumcarbonat Tablette
pH=7      Phosphatbindung: 120 mg/g
pH=7.5    Phosphatbindung: 135 mg/g
pH=8.0    Phosphatbindung: 160 mg/g

Calciumcarbonat (Hartgelatinekapsel)
pH=7.0    Phosphatbindung: 125 mg/g
pH=7.5    Phosphatbindung: 140 mg/g
pH=8.0    Phosphatbindung: 170 mg/g

Calciumsulfat . $2H_2O$ (Tablette)
pH=7.0    Phosphatbindung: 280 mg/g
pH=7.5    Phosphatbindung: 300 mg/g
pH=8.0    Phosphatbindung: 300 mg/g

Magnesiumcarbonat (Tablette)
pH=7.0    Phosphatbindung: 140 mg/g
pH=7.5    Phosphatbindung: 150 mg/g
pH=8.0    Phosphatbindung: 150 mg/g

Magnesiumoxid (Hartgelatinekapsel)
pH=7.0    Phosphatbindung: 160 mg/g
pH=7.5    Phosphatbindung: 160 mg/g
pH=8.0    Phosphatbindung: 180 mg/g.

Unter den gleichen Bedingungen beträgt die Phosphatbindung bei Aluminiumhydoxid (Aldrox® Tablette) 170 mg/g.

Beispiel 2

Zur Ermittlung der Oxalat-Bindungskapazität wurden zwei Reihen von Proben hergestellt:
a) 500 mg Oxalsäure in 1980 ml 0.1 M Tris-HCl
b) 500 mg Oxalsäure und 960 mg $K_2HPO_4$ in 190 ml 0.1 M Tris-HCl.

4

Zur Simulierung der pH-Verhältnisse in den einzelnen Darm-Abschnitten wurden innerhalb jeder Reihe Proben mit folgenden pH-Werten hergestellt:

pH=6,0; 6,5; 7,0; 7,5; 8,0.

Nach genauer Einstellung des pH-Wertes mit NaOH bzw. HCl wurde Puffer mit 200 ml aufgefüllt und jeder Probe 2 magensaftresistente Tabletten (a 500 mg Arzneistoff) bzw. magensaftresistente Hartgelatinekapseln (mit 500 mg Wirkstoff) bzw. magensaftresistente Weichgelatinekapseln (mit 500 mg Wirkstoff) zugesetzt.

Nach genauer Einstellung des pH-Wertes mit NaOH, HCl wurde mit Puffer auf 200 ml aufgefüllt. Die Ansätze wurden unter leichter Bewegung bei 37°C im Wasserbad inkubiert, und 60 Minuten jeweils 12 ml Aliquots entnommen, gebildetes Calciumoxalat, bzw. Magnesiumoxalat abzentrifugiert und vom Überstand jeweils 10 ml mit $KMnO_4$-Lösung (siehe Bestimmungsmethoden) titriert.

Ergebnisse:

### Calciumcarbonat (Tabletten, magensaftresistent)

| pH-Wert | Oxalatbindung (mg/g) |
|---------|----------------------|
| 6,0 | a) 180 mg<br>b) 150 mg |
| 6,5 | a) 170 mg<br>b) 145 mg |
| 7,0 | a) 165 mg<br>b) 145 mg |
| 7,5 | a) 165 mg<br>b) 140 mg |
| 8,0 | a) 165 mg<br>b) 140 mg |

### Calciumoxid (Weichgelatinekapseln, magensaftresistent)

| pH-Wert | Oxalatbindung (mg/g) |
|---------|----------------------|
| 6,0 | a) 150 mg<br>b) 130 mg |
| 6,5 | a) 160 mg<br>b) 140 mg |
| 7,0 | a) 160 mg<br>b) 140 mg |
| 7,5 | a) 165 mg<br>b) 140 mg |
| 8,0 | a) 170 mg<br>b) 150 mg |

### Magnesiumcarbonat (magensaftresistente Tabletten)

| pH-Wert | Oxalatbindung (mg/g) |
|---------|----------------------|
| 6,0 | a) 190 mg<br>b) 150 mg |
| 7,0 | a) 200 mg<br>b) 150 mg |
| 8,0 | a) 220 mg<br>b) 160 mg |

Magnesiumhydroxidcarbonat (magensaftresistente Hartgelatinekapseln)

| pH-Wert | Oxalatbindung (mg/g) |
|---------|----------------------|
| 6,0 | a) 180 mg<br>b) 140 mg |
| 6,5 | a) 180 mg<br>b) 150 mg |
| 7,0 | a) 200 mg<br>b) 160 mg |
| 8,0 | a) 210 mg<br>b) 180 mg |

**Patentansprüche für die Vertragsstaaten: DE GB FR NL IT BE SE CH/LI**

1. Arzneimittel zur Behandlung der Hyperphosphatämie und zur Prävention phosphat- und/oder oxalathaltiger Nierensteine, dadurch gekennzeichnet, daß es eine bei pH-Werten von 6 bis 9 schwerlösliche Calciumverbindung, nämlich Calciumcarbonat, Calciumhydroxid, Calciumoxid und/oder Calciumsulfat, gegebenenfalls zusammen mit üblichen Träger- und/oder Verdünnungsstoffen in magensaftresistenter Arzneiform enthält.

2. Arzneimittel nach Anspruch 1, dadurch gekennzeichnet, daß es in Form magensaftresistenter Tabletten, magensaftresistenter Hartgelatinekapseln, magensaftresistenter Weichgelatinekapseln oder als magensaftresistentes Granulat vorliegt.

3. Verwendung von mindestens einer bei pH-Werten von 6 bis 9 schwerlöslichen Calcium- und/oder Magnesiumverbindung zur Herstellung eines Arzneimittels in magensaftresistenter Arzneiform, zur Bekämpfung der Hyperphosphatämie und zur Prävention phosphat- und/oder oxalathaltiger Nierensteine.

4. Verwendung nach Anspruch 3, dadurch gekennzeichnet, daß man als eine bei einem pH-Wert von 6 bis 9 schwerlösliche Calciumverbindung Calciumcarbonat, Calciumhydroxid, Calciumoxid und/oder Calciumsulfat verwendet.

5. Verwendung nach Anspruch 3, dadurch gekennzeichnet, daß man als eine bei einem pH-Wert von 6 bis 9 schwerlösliche Magnesiumverbindung Magnesiumcarbonat, Magnesiumhydroxid-Carbonat, Magnesiumhydroxid und/oder Magnesiumoxid verwendet.

6. Verwendung von Calciumcarbonat zur Herstellung eines Arzneimittels in magensaftresistenter Form zur Bekämpfung der Hyperphosphatämie und zur Behandlung phosphathaltiger Nierensteine.

7. Verwendung von Magnesiumcarbonat, Magnesiumhydroxid-Carbonat, Magnesiumhydroxid und/oder Magnesiumoxid zur Herstellung eines Arzneimittels in magensaftresistenter Form zur Behandlung oxalathaltiger Nierensteine.

8. Verwendung von Calcium- und Magnesiumverbindungen, die bei pH-Werten von 6 bis 9 schwerlöslich sind, gemeinsam, zur Herstellung eines Arzneimittels in magesaftresistenter Form zur Bekämpfung oxalathaltiger Nierensteine.

**Patentansprüche für den Vertragsstaat: AT**

1. Verfahren zur Herstellung eines Arzneimittels zur Behandlung der Hyperphosphatämie und zur Prävention phosphat- und/oder oxalathaltiger Nierensteine, dadurch gekennzeichnet, daß man eine bei pH-Werten von 6 bis 9 schwerlösliche Calciumverbindung, nämlich Calciumcarbonat, Calciumhydroxid, Calciumoxid und/oder Calciumsulfat und übliche Träger- und/oder Verdünnungsstoffe vermischt und das Gemisch in an sich bekannter Weise in eine magensaftresistente Arzneiform überführt.

2. Verfahren nach Anspruch 1, dadurch gekennzeichnet, daß man das Mittel in Form magensaftresistenter Tabletten, magensaftresistenter Hartgelatinekapseln, magensaftresistenter Weichgelatinekapseln oder als magensaftresistentes Granulat herstellt.

3. Verwendung von mindestens einer bei pH-Werten von 6 bis 9 schwerlöslichen Calcium- und/oder Magnesiumverbindung zur Herstellung eines Arzneimittels in magensaftresistenter Arzneiform, zur Bekämpfung der Hyperphosphatämie und zur Prävention phosphat- und/oder oxalathaltiger Nierensteine.

4. Verwendung nach Anspruch 3, dadurch gekennzeichnet, daß man als eine bei einem pH-Wert von 6 bis 9 schwerlösliche Calciumverbindung Calciumcarbonat, Calciumhydroxid, Calciumoxid und/oder Calciumsulfat verwendet.

5. Verwendung nach Anspruch 3, dadurch gekennzeichnet, daß man als eine bei einem pH-Wert von 6 bis 9 schwerlösliche Magnesiumverbindung Magnesiumcarbonat, Magnesiumhydroxid-Carbonat, Magnesiumhydroxid und/oder Magnesiumoxid verwendet.

6. Verwendung von Calciumcarbonat zur Herstellung eines Arzneimittels in magensaftresistenter Form zur Bekämpfunmg der Hyperphosphatämie und zur Behandlung phosphathaltiger Nierensteine.

7. Verwendung von Magnesiumcarbonat, Magnesiumhydroxid-Carbonat, Magnesiumhydroxid und/oder Magnesiumoxid zur Herstellung eines Arzneimittels in magensaftresistenter Form zur Behandlung oxalathaltiger Nierensteine.

8. Verwendung von Calcium- und Magnesiumverbindungen, die bei pH-werten von 6 bis 9 schwerlöslich sind, gemeinsam, zur Herstellung eines Arzneimittels in magensaftresistenter Form zur Bekämpfung oxalathaltiger Nierensteine.

**Revendications pour les Etats contractants: DE GB FR NL IT BE SE CH/LI**

1. Médicament pour le traitement de l'hyperphosphatémie et pour la prévention de calculs rénaux contenant des phosphates et/ou des oxalates, caractérisé en ce qu'il renferme un composé de calcium difficilement soluble, à un pH de 6 à 9, tel le carbonate de calcium, l'hydroxyde de calcium, l'oxyde de calcium et/ou le sulfate de calcium, le cas échéant avec des composés de dilution et/ou vecteur habituels sous une forme médicamenteuse résistante dans l'estomac.

2. Médicament selon la revendication 1, caractérisé en ce qu'il se présente sous forme de comprimés, de capsules à gélatine dure, de capsules à gélatine malléable ou de granulat, tous résistants dans l'estomac.

3. Utilisation d'au moins un composé du calcium et/ou du magnésium difficilement soluble à un pH de 6 à 9, pour préparer un médicament sous une forme résistante dans l'estomac, pour combattre l'hyperphosphatémie et our prévenir la formation de calculs rénaux contenant des phosphates et/ou des oxalates.

4. Utilisation selon la revendication 3, caractérisée en ce qu'on utilise comme composé du calcium difficilement soluble à un pH de 6 à 9, le carbonate de calcium, l'hydroxyde de calcium, l'oxyde de calcium et/ou le sulfate de calcium.

5. Utilisation selon la revendication 3, caractérisée en ce qu'on utilise comme composé du magnésium difficilement soluble à un pH de 6 à 9, le carbonate de magnésium, l'hydroxyde de magnésium et/ou l'oxyde de magnésium.

6. Utilisation du carbonate de calcium pour la préparation d'un médicament sous une forme résistante dans l'estomac pour combattre l'hyperphosphatémie et pour traiter les calculs rénaux renfermant des phosphates.

7. Utilisation du carbonate de magnésium, du carbonate hydroxyde de magnésium, de l'hydoxyde de magnésium et/ou de l'oxyde de magnésium pour préparer un médicament sous forme resistante dans l'estomac pour traiter les calculs rénaux, renfermant des oxalates.

8. Utilisation des composés du calcium et du magnésium, difficilement solubles à un pH de 6 à 9, en combinaison, pour préparer un médicament sous une forme résistante dans l'estomac pour traiter les calculs rénaux renfermant des oxalates.

**Revendications pour l'Etat contractant: AT**

1. Procédé pour préparer un médicament pour le traitement de l'hyperphosphatémie et pour la prévention de calculs rénaux contenant des phosphates et/ou des oxalates, caractérisé en ce qu'on mélange à un pH de 6 à 9 un composé de calcium difficilement soluble, tel le carbonate de calcium, l'hydroxyde de calcium, l'oxyde de calcium et/ou le sulfate de calcium avec des composés de dilution et/ou vecteur habituels et en ce qu'on transforme le mélange de manière connue en une forme médicamenteuse résistante dans l'estomac.

2. Procédé selon la revendication 1, caractérisé en ce qu'on prépare le mélange sous forme de comprimés, de capsules à gélatine dure, de capsules à gélatine malléable ou de granulat, tous résistants dans l'estomac.

3. Utilisation d'au moins un composé du calcium et/ou du magnésium difficilement soluble à un pH de 6 à 9, pour préparer un médicament sous une forme résistante dans l'estomac, pour combattre l'hyperphosphatémie et pour prévenir la formation de calculs rénaux contenant des phosphates et/ou des oxalates.

4. Utilisation selon la revendication 3, caractérisée en ce qu'on utilise comme composé du calcium difficilement soluble à un pH de 6 à 9, le carbonate de calcium, l'hydroxyde de calcium, l'oxyde de calcium et/ou le sulfate de calcium.

5. Utilisation selon la revendication 3, caractérisée en ce qu'on utilise comme composé du magnésium difficilement soluble à un pH de 6 à 9, le carbonate de magnésium, l'hydroxyde de magnésium et/ou l'oxyde de magnésium.

6. Utilisation du carbonate de calcium pour la préparation d'un médicament sous une forme résistante dans l'estomac pour combattre l'hyperphosphatémie et pour traiter les calculs rénaux renfermant des phosphates.

7. Utilisation du carbonate de magnésium, du carbonate hydroxyde de magnésium, de l'hydroxyde de magnésium et/ou de l'oxyde de magnésium pour préparer un médicament sous forme résistante dans l'estomac pour traiter les calculs rénaux renfermant des oxalates.

8. Utilisation des composés du calcium et du magnésium difficilement solubles à un pH de 6 à 9, en combinaison, pour préparer un médicament sous une forme résistante dans l'estomac pour traiter les calculs rénaux renfermant des oxalates.

**Claims for the Contracting States: DE GB FR NL IT BE SE CH/LI**

1. A drug for treatment of hyperphosphataemia and prevention of phosphate-containing and/or oxalate-containing kidney stones, characterised in that it contains a calcium compound which is soluble with difficulty at pH 6—9, i.e. calcium carbonate, calcium hydroxide, calcium oxide and/or calcium sulphate, together if required with conventional excipients and/or diluents in a form resistant to gastric juices.

2. A drug according to Claim 1, characterised in that it is in the form of tablets or hard gelatin capsules or soft gelatin capsules or a granulate, all resistant to gastric juices.

3. Use of at least one calcium and/or magnesium compound which is soluble with difficulty at pH 6—9 for producing a drug in a form resistant to gastric juices, for treatment of hyperphosphataemia and prevention of phosphate-containing and/or oxalate-containing kidney stones.

4. Use according to Claim 3, characterised in that calcium carbonate, calcium hydroxide, calcium oxide and/or calcium sulphate is used as the calcium compound which is soluble with difficulty at a pH from 6 to 9.

5. Use according to Claim 3, characterised in that magnesium carbonate, magnesium hydroxide carbonate, magnesium hydroxide and/or magnesium oxide is used as the magnesium compound which is soluble with difficulty at a pH from 6 to 9.

6. Use of calcium carbonate for producing a drug in a form resistant to gastric juices and for treatment of hyperphosphataemia and treatment of phosphate-containing kidney stones.

7. Use of magnesium carbonate, magnesium hydroxide carbonate, magnesium hydroxide and/or magnesium oxide for producing a drug in a form resistant to gastric juices and for treatment of oxalate-containing kidney stones.

8. Use of calcium and magnesium compounds which are soluble with difficulty at a pH from 6 to 9 in combination for producing a drug in a form resistant to gastric juices and for counteracting of oxalate-containing kidney stones.

**Claims for the Contracting State: AT**

1. A method of producing a drug for treatment of hyperphosphataemia and for prevention of phosphate containing and/or oxalate-containing kidney stones, characterised in that a calcium compound soluble with difficulty at a pH from 6 to 9, i.e. calcium carbonate, calcium hydroxide, calcium oxide and/or calcium sulphate is mixed with conventional excipients and/or diluents and the mixture is converted in known manner into a form of drug resistant to gastric juices.

2. A method according to Claim 1, characterised in that the drug is produced in the form of tablets or hard gelatin capsules or soft gelatin capsules or a granulate, all resistant to gastric juices.

3. Use of at least one calcium and/or magnesium compound which is soluble with difficulty at pH 6—9 for producing a drug in a form resistant to gastric juices, for treatment of hyperphosphataemia and prevention of phosphate-containing and/or oxalate-containing kidney stones.

4. Use according to Claim 3, characterised in that calcium carbonate, calcium hydroxide, calcium oxide and/or calcium sulphate is used as the calcium compound which is soluble with difficulty at a pH from 6 to 9.

5. Use according to Claim 3, characterised in that magnesium carbonate, magnesium hydroxide carbonate, magnesium hydroxide and/or magnesium oxide is used as the magnesium compound which is soluble with difficulty at a pH from 6 to 9.

6. Use of calcium carbonate for producing a drug in a form resistant to gastric juices and for treatment of hyperphosphataemia and treatment of phosphate-containing kidney stones.

7. Use of magnesium carbonate, magnesium hydroxide carbonate, magnesium hydroxide and/or magnesium oxide for producing a drug in a form resistant to gastric juices and for treatment of oxalate-containing kidney stones.

8. Use of calcium and magnesium compounds which are soluble with difficulty at a pH from 6 to 9 in combination for producing a drug in a form resistant to gastric juices and for counteracting oxalate-containing kidney stones.